# EUROPEAN PATENT APPLICATION

(11) **EP 3 158 996 A1**
(43) Date of publication of application: **26.04.2017**
(21) Application number: 15809724.6
(22) Date of filing: 11.06.2015
(51) Int. Cl.: A61K 31/495, A61K 31/496, A61K 31/4985, A61K 36/48, A61K 36/82, A61K 38/00, A61P 3/10, A61P 5/50

(54) **GLUCOSE METABOLISM AMELIORATING AGENT**

(30) Priority: 20.06.2014 JP 2014127769
(71) Applicant: Suntory Holdings Limited, Osaka-shi, Osaka 530-8203 (JP)
(72) Inventor: SUZUKI, Toshihide, Soraku-gun Kyoto 619-0284 (JP); YAMAMOTO, Kenji, Soraku-gun Kyoto 619-0284 (JP); BEPPU, Yoshinori, Soraku-gun Kyoto 619-0284 (JP); WATANABE, Hiroshi, Soraku-gun Kyoto 619-0284 (JP)
(74) Representative: Vossius & Partner Patentanwälte Rechtsanwälte mbB
(86) International application number: PCT/JP2015/066817
(87) International publication number: WO 2015/194447

(57) **Abstract**

Provided is a safe carbohydrate metabolism-ameliorating agent having an excellent carbohydrate metabolism-ameliorating action, and a safe GLP-1 secretion accelerator having an excellent GLP-1 secretion-accelerating action. The carbohydrate metabolism-ameliorating agent or GLP-1 secretion accelerator according to the present invention, which contains a specific cyclic dipeptide or a salt thereof as an active ingredient, is advantageous in that it has an excellent carbohydrate metabolism-ameliorating action, and that it is safe and can be ingested for a long period.

## Description

### TECHNICAL FIELD

The present invention relates to a carbohydrate metabolism-ameliorating agent. More specifically, the present invention relates to a carbohydrate metabolism-ameliorating agent containing a cyclic dipeptide including amino acids as constituent units as an active ingredient, a GLP-1 secretion accelerator containing the cyclic dipeptide as an active ingredient, and use of the cyclic dipeptide for ameliorating carbohydrate metabolism.

### BACKGROUND ART

"Dipeptides", which are each composed of two amino acids bonded to each other, have been paid attention as functional substances. Dipeptides can be provided with physical properties or novel functions that are not possessed by simple amino acids and are expected as materials having application ranges broader than those of amino acids. In particular, diketopiperazines, which are cyclic dipeptides, are known to have various physiological activities, and demands for diketopiperazines are predicted to increase in the medical and pharmacological fields.

For example, Patent Literature 1 reports that cyclic dipeptides having 2,5-diketopiperazine structure have an antidepressant action, a learning motivation-improving action, etc. Non Patent Literature 1 discloses that the cyclic dipeptide Cyclo(His-Pro) has various physiological activities including central nervous system actions such as decrease in body temperature and appetite suppression and hormone-like actions such as suppression of prolactin secretion and acceleration of growth hormone secretion, and the literature also reports that the cyclic dipeptide Cyclo(Leu-Gly) has a memory function-improving action and the cyclic dipeptide Cyclo(Asp-Pro) has a suppressing action on preference for fat. Non Patent Literature 2 reports cyclic dipeptides having an antibacterial action or an antioxidant action.

Non Patent Literature 3 reports that the cyclic dipeptide Cyclo(Trp-Pro) has an anticancer action, the cyclic dipeptides Cyclo(His-Pro) and Cyclo(Gly-Pro) have an antibacterial action, the cyclic dipeptide Cyclo(His-Pro) has a neuroprotective action, the cyclic dipeptide Cyclo(Gly-Pro) has a memory function-improving action, and the cyclic dipeptides Cyclo(Tyr-Pro) and Cyclo(Phe-Pro) have a biological herbicide action. It is reported that cyclo-histidyl-proline (Cyclo(His-Pro)), which is one of cyclic dipeptides and obtained by treating a yeast suspension with protease, increases glucose tolerance and Cyclo(His-Pro) has an antioxidant activity (Non Patent Literature 4).

The glucagon-like peptide (GLP-1), which is known as a weight-loss hormone, is an incretin consisting of 30 or 31 amino acids, and is released from enteroendocrine L cells in response to fats and oils, uptake of carbohydrates, and proteins derived from diet. It has been found that release of the peptide hormone decreases in individuals with Type II diabetes and acceleration of GLP-1 release in Type II diabetes is considered to be effective for treatment of diabetes and other related diseases (Non Patent Literature 5). In addition, the GLP-1 is known to have a function of appetite suppression through increase in insulin secretion in response to uptake of carbohydrates (uptake of glucose) to work on a specific area in the brain in normal condition (Non Patent Literature 6).

### CITATION LIST

### PATENT LITERATURE

Patent Literature 1: National Publication of International Patent Application No. 2012-517998

### NON PATENT LITERATURE

Non Patent Literature 1: Peptides, 16(1), 151-164 (1995)
Non Patent Literature 2: Bioscience & Industry, 60(7), 454-457 (2002)
Non Patent Literature 3: Chemical Reviews, 112, 3641-3716 (2012)
Non Patent Literature 4: J of Food Science, vol 76(2) 2011
Non Patent Literature 5: Nauck et al., 1993, J Clin Invest. 1993 Jan; 91(1):301-7
Non Patent Literature 6: Zander et al., 2002, Lancet, 359, 824-830 (2002)

### SUMMARY OF INVENTION

### TECHNICAL PROBLEM

Although cyclic dipeptides are reported to have a physiological activity, they possibly have still unknown functions.

It is an object of the present invention to provide a safe carbohydrate metabolism-ameliorating agent having an excellent carbohydrate metabolism-ameliorating action, and a safe GLP-1 secretion accelerator having an excellent GLP-1 secretion-accelerating action.

### SOLUTION TO PROBLEM

The present inventors, who have diligently studied the action of cyclic dipeptides, have found that specific cyclic dipeptides have a significant carbohydrate metabolism-ameliorating action, and further found that the carbohydrate metabolism-ameliorating action of the specific cyclic dipeptides is due to a GLP-1 secretion-accelerating action, and have arrived at the completion of the present invention. Specifically, the present invention provides the following aspects:
1) A carbohydrate metabolism-ameliorating agent containing a cyclic dipeptide including amino acids as constituent units or a salt thereof as an active ingredient, wherein the cyclic dipeptide or salt thereof is one or two or more cyclic dipeptides selected from the group consisting of cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, cyclo-isoleucyl-lysine, cyclo-glutaminyl-leucine, cyclo-isoleucyl-threonine, cyclo-threonyl-threonine, cyclo-methionyl-threonine, cyclo-alanyl-cysteine, cyclo-glycyl-cysteine, cyclo-aspartyl-serine, cyclo-arginyl-aspartic acid, cyclo-glycyl-tryptophan, cyclo-histidyl-phenylalanine, cyclo-aspartyl-leucine, cyclo-isoleucyl-histidine, cyclo-seryl-leucine, cyclo-isoleucyl-aspartic acid, cyclo-seryl-cysteine, cyclo-phenylalanyl-tryptophan, cyclo-alanyl-leucine, cyclo-glutaminyl-histidine, cyclo-arginyl-valine, cyclo-glutamyl-leucine, cyclo-leucyl-tryptophan, cyclo-tryptophanyl-tryptophan, cyclo-L-alanyl-proline, cyclo-methionyl-arginine, cyclo-lysyl-phenylalanine, cyclo-phenylalanyl-phenylalanine, cyclo-tryptophanyl-tyrosine, cyclo-asparaginyl-valine, cyclo-glutaminyl-isoleucine, cyclo-alanyl-serine, cyclo-methionyl-histidine, cyclo-methionyl-proline, cyclo-arginyl-leucine, cyclo-methionyl-glutamic acid, cyclo-methionyl-alanine, cyclo-isoleucyl-glutamic acid, cyclo-isoleucyl-serine, cyclo-valyl-serine, cyclo-methionyl-glycine, cyclo-valyl-threonine, cyclo-valyl-aspartic acid, cyclo-glycyl-proline, cyclo-leucyl-proline, cyclo-glutaminyl-glycine, cyclo-tryptophanyl-lysine, cyclo-glutaminyl-phenylalanine, cyclo-lysyl-glycine, cyclo-seryl-lysine, cyclo-valyl-lysine, cyclo-asparaginyl-lysine, cyclo-histidyl-histidine, cyclo-threonyl-histidine, cyclo-aspartyl-histidine, cyclo-asparaginyl-histidine, cyclo-arginyl-serine, cyclo-asparaginyl-methionine, cyclo-glutaminyl-methionine, cyclo-tryptophanyl-arginine, cyclo-asparaginyl-arginine, cyclo-asparaginyl-proline, and cyclo-arginyl-arginine or salts thereof.
2) The carbohydrate metabolism-ameliorating agent according to aspect 1), wherein the cyclic dipeptide or salt thereof is three or more cyclic dipeptides selected from the group defined in aspect 1) or salts thereof.
3) A GLP-1 secretion accelerator containing a cyclic dipeptide including amino acids as constituent units or a salt thereof as an active ingredient, wherein the cyclic dipeptide or salt thereof is one or two or more cyclic dipeptides selected from the group consisting of cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, cyclo-isoleucyl-lysine, cyclo-glutaminyl-leucine, cyclo-isoleucyl-threonine, cyclo-threonyl-threonine, cyclo-methionyl-threonine, cyclo-alanyl-cysteine, cyclo-glycyl-cysteine, cyclo-aspartyl-serine, cyclo-arginyl-aspartic acid, cyclo-glycyl-tryptophan, cyclo-histidyl-phenylalanine, cyclo-aspartyl-leucine, cyclo-isoleucyl-histidine, cyclo-seryl-leucine, cyclo-isoleucyl-aspartic acid, cyclo-seryl-cysteine, cyclo-phenylalanyl-tryptophan, cyclo-alanyl-leucine, cyclo-glutaminyl-histidine, cyclo-arginyl-valine, cyclo-glutamyl-leucine, cyclo-leucyl-tryptophan, cyclo-tryptophanyl-tryptophan, cyclo-L-alanyl-proline, cyclo-methionyl-arginine, cyclo-lysyl-phenylalanine, cyclo-phenylalanyl-phenylalanine, cyclo-tryptophanyl-tyrosine, cyclo-asparaginyl-valine, cyclo-glutaminyl-isoleucine, cyclo-alanyl-serine, cyclo-methionyl-histidine, cyclo-methionyl-proline, cyclo-arginyl-leucine, cyclo-methionyl-glutamic acid, cyclo-methionyl-alanine, cyclo-isoleucyl-glutamic acid, cyclo-isoleucyl-serine, cyclo-valyl-serine, cyclo-methionyl-glycine, cyclo-valyl-threonine, and cyclo-valyl-aspartic acid, cyclo-glycyl-proline, cyclo-leucyl-proline, cyclo-glutaminyl-glycine, cyclo-tryptophanyl-lysine, cyclo-glutaminyl-phenylalanine, cyclo-lysyl-glycine, cyclo-seryl-lysine, cyclo-valyl-lysine, cyclo-asparaginyl-lysine, cyclo-histidyl-histidine, cyclo-threonyl-histidine, cyclo-aspartyl-histidine, cyclo-asparaginyl-histidine, cyclo-arginyl-serine, cyclo-asparaginyl-methionine, cyclo-glutaminyl-methionine, cyclo-tryptophanyl-arginine, cyclo-asparaginyl-arginine, cyclo-asparaginyl-proline, and cyclo-arginyl-arginine or salts thereof.
4) The GLP-1 secretion accelerator according to aspect 3), wherein the cyclic dipeptide or salt thereof is three or more cyclic dipeptides selected from the group defined in 3) or salts thereof.
5) The GLP-1 secretion accelerator according to any one of aspects 1) to 4), wherein the cyclic dipeptide including amino acids as constituent units is derived from soybean.
6) The GLP-1 secretion accelerator according to any one of aspects 1) to 4), wherein the cyclic dipeptide including amino acids as constituent units is derived from tea.
7) Use of a cyclic dipeptide including amino acids as constituent units or a salt thereof for ameliorating carbohydrate metabolism, wherein the cyclic dipeptide or salt thereof is one or two or more cyclic dipeptides selected from the group consisting of cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, cyclo-isoleucyl-lysine, cyclo-glutaminyl-leucine, cyclo-isoleucyl-threonine, cyclo-threonyl-threonine, cyclo-methionyl-threonine, cyclo-alanyl-cysteine, cyclo-glycyl-cysteine, cyclo-aspartyl-serine, cyclo-arginyl-aspartic acid, cyclo-glycyl-tryptophan, cyclo-histidyl-phenylalanine, cyclo-aspartyl-leucine, cyclo-isoleucyl-histidine, cyclo-seryl-leucine, cyclo-isoleucyl-aspartic acid, cyclo-seryl-cysteine, cyclo-phenylalanyl-tryptophan, cyclo-alanyl-leucine, cyclo-glutaminyl-histidine, cyclo-arginyl-valine, cyclo-glutamyl-leucine, cyclo-leucyl-tryptophan, cyclo-tryptophanyl-tryptophan, cyclo-L-alanyl-proline, cyclo-methionyl-arginine, cyclo-lysyl-phenylalanine, cyclo-phenylalanyl-phenylalanine, cyclo-tryptophanyl-tyrosine, cyclo-asparaginyl-valine, cyclo-glutaminyl-isoleucine, cyclo-alanyl-serine, cyclo-methionyl-histidine, cyclo-methionyl-proline, cyclo-arginyl-leucine, cyclo-methionyl-glutamic acid, cyclo-methionyl-alanine, cyclo-isoleucyl-glutamic acid, cyclo-isoleucyl-serine, cyclo-valyl-serine, cyclo-methionyl-glycine, cyclo-valyl-threonine, and cyclo-valyl-aspartic acid, cyclo-glycyl-proline, cyclo-leucyl-proline, cyclo-glutaminyl-glycine, cyclo-tryptophanyl-lysine, cyclo-glutaminyl-phenylalanine, cyclo-lysyl-glycine, cyclo-seryl-lysine, cyclo-valyl-lysine, cyclo-asparaginyl-lysine, cyclo-histidyl-histidine, cyclo-threonyl-histidine, cyclo-aspartyl-histidine, cyclo-asparaginyl-histidine, cyclo-arginyl-serine, cyclo-asparaginyl-methionine, cyclo-glutaminyl-methionine, cyclo-tryptophanyl-arginine, cyclo-asparaginyl-arginine, cyclo-asparaginyl-proline, and cyclo-arginyl-arginine or salts thereof.
8) The use according to aspect 7), wherein the cyclic dipeptide or salt thereof is three or more cyclic dipeptides selected from the group defined in aspect 7) or salts thereof.
9) The use according to aspect 7) or 8), wherein the cyclic dipeptide including amino acids as constituent units is derived from soybean.
10) The use according to aspect 7) or 8), wherein the cyclic dipeptide including amino acids as constituent units is derived from tea.

### ADVANTAGEOUS EFFECTS OF INVENTION

The carbohydrate metabolism-ameliorating agent according to the present invention is advantageous in that it has an excellent carbohydrate metabolism-ameliorating action, and that it is safe and can be ingested for a long period.

### BRIEF DESCRIPTION OF DRAWINGS

Fig. 1 shows the plasma active GLP-1 level after administration of a heat-treated soybean peptide.
Fig. 2 shows the results of a glucose-loading test after administration of a heat-treated soybean peptide and a soybean peptide.
Fig. 3 shows the results of a glucose-loading test after administration of a heat-treated tea peptide.

### DESCRIPTION OF EMBODIMENTS

One aspect of the present invention is a carbohydrate metabolism-ameliorating agent containing a cyclic dipeptide including amino acids as constituent units or a salt thereof as an active ingredient. Throughout the specification, cyclic dipeptides or salts thereof are occasionally referred to as cyclic dipeptides in a collective and simple manner.

The carbohydrate metabolism-ameliorating agent according to the present invention contains one or two or more cyclic dipeptides selected from group A consisting of cyclo-threonyl-tyrosine [Cyclo(Thr-Tyr)], cyclo-valyl-tyrosine [Cyclo(Val-Tyr)], cyclo-glutaminyl-tyrosine [Cyclo(Gln-Tyr)], cyclo-asparaginyl-isoleucine [Cyclo(Asn-Ile)], cyclo-arginyl-proline [Cyclo(Arg-Pro)], cyclo-asparaginyl-glycine [Cyclo(Asn-Gly)], cyclo-methionyl-valine [Cyclo(Met-Val)], cyclo-glutamyl-cysteine [Cyclo(Glu-Cys)], cyclo-seryl-glutamic acid [Cyclo(Ser-Glu)], cyclo-isoleucyl-lysine [Cyclo(Ile-Lys)], cyclo-glutaminyl-leucine [Cyclo(Gln-Leu)], cyclo-isoleucyl-threonine [Cyclo(Ile-Thr)], cyclo-threonyl-threonine [Cyclo(Thr-Thr)], cyclo-methionyl-threonine [Cyclo(Met-Thr)], cyclo-alanyl-cysteine [Cyclo(Ala-Cys)], cyclo-glycyl-cysteine [Cyclo(Gly-Cys)], cyclo-aspartyl-serine [Cyclo(Asp-Ser)], cyclo-arginyl-aspartic acid [Cyclo(Arg-Asp)], cyclo-glycyl-tryptophan [Cyclo(Gly-Trp)], cyclo-histidyl-phenylalanine [Cyclo(His-Phe)], cyclo-aspartyl-leucine [Cyclo(Asp-Leu)], cyclo-isoleucyl-histidine [Cyclo(Ile-His)], cyclo-seryl-leucine [Cyclo(Ser-Leu)], cyclo-isoleucyl-aspartic acid [Cyclo(Ile-Asp)], cyclo-seryl-cysteine [Cyclo(Ser-Cys)], cyclo-phenylalanyl-tryptophan [Cyclo(Phe-Trp)], cyclo-alanyl-leucine [Cyclo(Ala-Leu)], cyclo-glutaminyl-histidine [Cyclo(Gln-His)], cyclo-arginyl-valine [Cyclo(Arg-Val)], cyclo-glutamyl-leucine [Cyclo(Glu-Leu)], cyclo-leucyl-tryptophan [Cyclo(Leu-Trp)], cyclo-tryptophanyl-tryptophan [Cyclo(Trp-Trp)], cyclo-L-alanyl-proline [Cyclo(L-Ala-Pro)], cyclo-methionyl-arginine [Cyclo(Met-Arg)], cyclo-lysyl-phenylalanine [Cyclo(Lys-Phe)], cyclo-phenylalanyl-phenylalanine [Cyclo(Phe-Phe)], cyclo-tryptophanyl-tyrosine [Cyclo(Trp-Tyr)], cyclo-asparaginyl-valine [Cyclo(Asn-Val)], cyclo-glutaminyl-isoleucine [Cyclo(Gln-Ile)], cyclo-alanyl-serine [Cyclo(Ala-Ser)], cyclo-methionyl-histidine [Cyclo(Met-His)], cyclo-methionyl-proline [Cyclo(Met-Pro)], cyclo-arginyl-leucine [Cyclo(Arg-Leu)], cyclo-methionyl-glutamic acid [Cyclo(Met-Glu)], cyclo-methionyl-alanine [Cyclo(Met-Ala)], cyclo-isoleucyl-glutamic acid [Cyclo(Ile-Glu)], cyclo-isoleucyl-serine [Cyclo(Ile-Ser)], cyclo-valyl-serine [Cyclo(Val-Ser)], cyclo-methionyl-glycine [Cyclo(Met-Gly)], cyclo-valyl-threonine [Cyclo(Val-Thr)], cyclo-valyl-aspartic acid [Cyclo(Val-Asp)], cyclo-glycyl-proline [Cyclo(Gly-Pro)], cyclo-leucyl-proline [Cyclo(Leu-Pro)], cyclo-glutaminyl-glycine [Cyclo(Gln-Gly)], cyclo-tryptophanyl-lysine [Cyclo(Trp-Lys)], cyclo-glutaminyl-phenylalanine [Cyclo(Gln-Phe)], cyclo-lysyl-glycine [Cyclo(Lys-Gly)], cyclo-seryl-lysine [Cyclo(Ser-Lys)], cyclo-valyl-lysine [Cyclo(Val-Lys)], cyclo-asparaginyl-lysine [Cyclo(Asn-Lys)], cyclo-histidyl-histidine [Cyclo(His-His)], cyclo-threonyl-histidine [Cyclo(Thr-His)], cyclo-aspartyl-histidine [Cyclo(Asp-His)], cyclo-asparaginyl-histidine [Cyclo(Asn-His)], cyclo-arginyl-serine [Cyclo(Arg-Ser)], cyclo-asparaginyl-methionine [Cyclo(Asn-Met)], cyclo-glutaminyl-methionine [Cyclo(Gln-Met)], cyclo-tryptophanyl-arginine [Cyclo(Trp-Arg)], cyclo-asparaginyl-arginine [Cyclo(Asn-Arg)], cyclo-asparaginyl-proline [Cyclo(Asn-Pro)], and cyclo-arginyl-arginine [Cyclo(Arg-Arg)] or salts thereof as an active ingredient. Alternatively, the carbohydrate metabolism-ameliorating agent according to the present invention contains three or more cyclic dipeptides or salts thereof selected from the above group of cyclic dipeptides or salts thereof as an active ingredient. Throughout the specification, the order of amino acids in a cyclic dipeptide may be inverse as long as the constitution is unchanged, and for example, Cyclo(Trp-Tyr) and Cyclo(Tyr-Trp) represent an identical cyclic dipeptide.

The carbohydrate metabolism-ameliorating agent according to the present invention is only required to contain one or two or more cyclic dipeptides of the above-mentioned 71 cyclic dipeptides or salts thereof as an active ingredient. Among them, one or two or more cyclic dipeptides selected from group B consisting of cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, cyclo-isoleucyl-lysine, cyclo-glutaminyl-leucine, cyclo-isoleucyl-threonine, cyclo-threonyl-threonine, cyclo-methionyl-threonine, cyclo-alanyl-cysteine, cyclo-glycyl-cysteine, cyclo-aspartyl-serine, cyclo-arginyl-aspartic acid, cyclo-glycyl-tryptophan, cyclo-histidyl-phenylalanine, cyclo-aspartyl-leucine, cyclo-isoleucyl-histidine, cyclo-seryl-leucine, cyclo-isoleucyl-aspartic acid, cyclo-seryl-cysteine, cyclo-phenylalanyl-tryptophan, cyclo-alanyl-leucine, cyclo-glutaminyl-histidine, cyclo-arginyl-valine, cyclo-glutamyl-leucine, cyclo-leucyl-tryptophan, cyclo-tryptophanyl-tryptophan, cyclo-L-alanyl-proline, cyclo-methionyl-arginine, cyclo-lysyl-phenylalanine, cyclo-phenylalanyl-phenylalanine, cyclo-tryptophanyl-tyrosine, cyclo-asparaginyl-valine, and cyclo-glutaminyl-isoleucine or salts thereof are preferably at least contained. More preferably, one or two or more cyclic dipeptides selected from group C consisting of cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, and cyclo-isoleucyl-lysine or salts thereof are at least contained.

Let two amino acids constituting the cyclic dipeptide according to the present invention be amino acid A and amino acid B. The cyclic dipeptide according to the present invention has a structure in which the carboxy group of amino acid A and the amino group of amino acid B have undergone dehydration condensation and the amino group of amino acid A and the carboxy group of amino acid B have undergone dehydration condensation. At least one of the constituent amino acids is preferably a basic amino acid, and more preferably arginine, but is not limited thereto.

The carbohydrate metabolism-ameliorating agent according to the present invention can accelerate GLP-1 secretion to ameliorate carbohydrate metabolism due to a feature of containing the cyclic dipeptide or salt thereof in an effective amount, and thus can be suitably used for diseases for which amelioration of carbohydrate metabolism is required. Examples of such diseases include diabetes and obesity, and the composition according to the present invention is effective for prevention and treatment of them.

The carbohydrate metabolism-ameliorating agent according to the present invention can be provided, for example, with a label indicating that this product is for prevention and/or amelioration of hyperglycemia and obesity, and is thus extremely useful for individuals with a high blood glucose level, slightly obese individuals, individuals with a tendency of metabolic syndrome, or the like.

One aspect of the present invention is a GLP-1 secretion accelerator containing a cyclic dipeptide including amino acids as constituent units or a salt thereof as an active ingredient. Most of the above descriptions regarding the carbohydrate metabolism-ameliorating agent is also applied to the GLP-1 secretion accelerator.

The carbohydrate metabolism-ameliorating agent according to the present invention is only required to contain one or two or more cyclic dipeptides of the above-mentioned 71 cyclic dipeptides or salts thereof in an amount which allows the effect to be exerted. The content of the cyclic dipeptide or salt thereof can be measured in accordance with a known method, for example, by using LC-MS/MS.

Throughout the specification, a salt of a cyclic dipeptide refers to any pharmacologically acceptable salt (including inorganic salts and organic salts), and examples thereof include sodium salts, potassium salts, calcium salts, magnesium salts, ammonium salts, hydrochlorides, sulfates, nitrates, phosphates, and organic acid salts (acetate, citrates, maleates, malates, oxalates, lactates, succinate, fumarates, propionates, formates, benzoates, picrates, and benzenesulfonates) of the cyclic dipeptide.

Cyclic dipeptides used in the present invention can be prepared by using a method known in the art. For example, a cyclic dipeptide may be produced by using chemical synthesis, an enzymatic method, or microbial fermentation, or may be synthesized by using a dehydration and cyclization reaction of a linear peptide, or may be prepared in accordance with a method described in Japanese Patent Laid-Open No. 2003-252896 or J. Peptide Sci., 10, 737-737 (2004). For example, a heat-treated product obtained by subjecting a soybean peptide or tea peptide obtained through enzyme treatment or heat treatment to further heat treatment can be preferably used. In other words, the cyclic dipeptide including amino acids as constituent units in the present invention may be derived from soybean or tea.

Specifically, a heat-treated product obtained by subjecting a solution containing a soybean peptide to heat treatment can be prepared, for example, by dissolving a soybean peptide in water to a concentration of 20 to 500 g/mL and heating the resultant under a condition of 40 to 150°C for 5 minutes to 120 hours. As desired, the heat-treated product obtained may be subjected to a process such as filtration, centrifugation, concentration, ultrafiltration, freeze-drying, and pulverization.

Specifically, a heat-treated product obtained by subjecting a solution containing a tea peptide to heat treatment can be prepared, for example, by dissolving a tea peptide in water to a concentration of 20 to 500 g/mL and heating the resultant under a condition of 40 to 150°C for 5 minutes to 120 hours. As desired, the heat-treated product obtained may be subjected to a process such as filtration, centrifugation, concentration, ultrafiltration, freeze-drying, and pulverization.

Those skilled in the art could easily prepare a salt of a cyclic dipeptide by using a method known in the art.

The cyclic dipeptide or salt thereof obtained as described above can be used for accelerating GLP-1 secretion to ameliorate carbohydrate metabolism. Accordingly, the present invention further provides a GLP-1 secretion accelerator containing the cyclic dipeptide or salt thereof according to the present invention as an active ingredient.

The carbohydrate metabolism-ameliorating agent or a GLP-1 secretion accelerator according to the present invention can be ingested by using an appropriate method in accordance with its form. The method of ingestion is not particularly limited and may be any method which allows the cyclic dipeptide or salt thereof according to the present invention to be transferred into the circulating blood. Throughout the specification, ingestion includes all modes of eating, administration, and drinking.

For example, a solvent, a dispersant, an emulsifier, a buffer, a stabilizer, a diluent, a binder, a disintegrator, a lubricant, etc., can be added to a raw material containing the cyclic dipeptide or salt thereof of the carbohydrate metabolism-ameliorating agent according to the present invention, as desired, to formulate a solid preparation such as a tablet, a granule, a powder(sanzai), a powder(funmatsuzai),and a capsule, or a solution such as a common solution, a suspension, and an emulsion by using a known method. Each of these compositions can be ingested as it is together with water or the like. In addition, the carbohydrate metabolism-ameliorating agent according to the present invention can be prepared in a form capable of being mixed easily (e.g., form of a powder or a granule) and used for a raw material of a drug.

The content of the cyclic dipeptide or salt thereof in the above form is not particularly limited and may be any content such that a desired effect of the present invention is exerted in view of form of administration, an administration method, etc. For example, the total amount of cyclic dipeptides including amino acids as constituent units of group A or salts thereof, more preferably the total content of cyclic dipeptides of group B or salts thereof, further preferably the total content of cyclic dipeptides of group C or salts thereof is preferably 0.2 × 10 ppm or more, more preferably 0.2 × 10² ppm or more, further preferably 0.4 × 10² ppm or more and preferably 1.0 × 10⁶ ppm or less, more preferably 1.0 × 10⁵ ppm or less, further preferably 0.5 × 10⁵ ppm or less. In the case of a solution or the like, the total amount of cyclic dipeptides including amino acids as constituent units of group A or salts thereof, more preferably the total content of cyclic dipeptides of group B or salts thereof, further preferably the total content of cyclic dipeptides of group C or salts thereof may be preferably 0.2 × 10⁻² mg/100 mL or more, more preferably 0.2 × 10⁻¹ mg/100 mL or more, further preferably 0.2 mg/100 mL or more and preferably 1.0 × 10³ mg/100 mL or less, more preferably 1.0 × 10² mg/100 mL or less, further preferably 0.5 × 10² mg/100 mL or less. The fraction of the total amount of cyclic dipeptides of group B or group C, each of which has a high GLP-1 secretion-accelerating action, in the cyclic dipeptide in Embodiment 1 is preferably 5% by weight or more, more preferably 10% by weight or more, and further preferably 15% by weight or more, from the viewpoint of a GLP-1 secretion-accelerating effect. The upper limit is not particularly limited, but preferably 90% by weight or less and more preferably 60% by weight or less. For example, the content of each cyclic dipeptide or salt thereof is as follows:
(1) cyclo-threonyl-tyrosine or a salt thereof, content: 0.40 × 10 to 0.70 × 10⁵ ppm, preferably 0.40 × 10² to 0.70 × 10⁴ ppm.
(2) cyclo-valyl-tyrosine or a salt thereof, content: 0.30 × 10 to 0.50 × 10⁵ ppm, preferably 0.30 × 10² to 0.50 × 10⁴ ppm.
(3) cyclo-glutaminyl-tyrosine or a salt thereof, content: 0.20 × 10 to 0.40 × 10⁵ ppm, preferably 0.20 × 10² to 0.40 × 10⁴ ppm.
(4) cyclo-asparaginyl-isoleucine or a salt thereof, content: 0.40 × 10 to 0.70 × 10⁵ ppm, preferably 0.40 × 10² to 0.70 × 10⁴ ppm.
(5) cyclo-arginyl-proline or a salt thereof, content: 0.40 × 10 to 0.70 × 10⁵ ppm, preferably 0.40 × 10² to 0.70 × 10⁴ ppm.
(6) cyclo-asparaginyl-glycine or a salt thereof, content: 0.20 × 10 to 0.50 × 10⁵ ppm, preferably 0.20 × 10² to 0.50 × 10⁴ ppm.
(7) cyclo-methionyl-valine or a salt thereof, content: 0.40 × 10 to 0.70 × 10⁵ ppm, preferably 0.40 × 10² to 0.70 × 10⁴ ppm.
(8) cyclo-glutamyl-cysteine or a salt thereof, content: 0.40 × 10 to 0.70 × 10⁵ ppm, preferably 0.40 × 10² to 0.70 × 10⁴ ppm.
(9) cyclo-seryl-glutamic acid or a salt thereof, content: 0.40 × 10 to 0.70 × 10⁵ ppm, preferably 0.40 × 10² to 0.70 × 10⁴ ppm.
(10) cyclo-isoleucyl-lysine or a salt thereof, content: 0.50 × 10 to 0.90 × 10⁵ ppm, preferably 0.50 × 10² to 0.90 × 10⁴ ppm.
(11) cyclo-glutaminyl-leucine or a salt thereof, content: 0.80 × 10 to 1.30 × 10⁵ ppm, preferably 0.80 × 10² to 1.30 × 10⁴ ppm.
(12) cyclo-isoleucyl-threonine or a salt thereof, content: 0.20 × 10 to 0.50 × 10⁵ ppm, preferably 0.20 × 10² to 0.50 × 10⁴ ppm.
(13) cyclo-threonyl-threonine or a salt thereof, content: 0.30 × 10 to 0.50 × 10⁵ ppm, preferably 0.30 × 10² to 0.50 × 10⁴ ppm.
(14) cyclo-methionyl-threonine or a salt thereof, content: 0.40 × 10 to 0.70 × 10⁵ ppm, preferably 0.40 × 10² to 0.70 × 10⁴ ppm.
(15) cyclo-alanyl-cysteine or a salt thereof, content: 0.40 × 10 to 0.70 × 10⁵ ppm, preferably 0.40 × 10² to 0.70 × 10⁴ ppm.
(16) cyclo-glycyl-cysteine or a salt thereof, content: 0.40 × 10 to 0.70 × 10⁵ ppm, preferably 0.40 × 10² to 0.70 × 10⁴ ppm.
(17) cyclo-aspartyl-serine or a salt thereof, content: 1.00 × 10 to 1.60 × 10⁵ ppm, preferably 1.00 × 10² to 1.60 × 10⁴ ppm.
(18) cyclo-arginyl-aspartic acid or a salt thereof, content: 0.90 × 10 to 1.50 × 10⁵ ppm, preferably 0.90 × 10² to 1.50 × 10⁴ ppm.
(19) cyclo-glycyl-tryptophan or a salt thereof, content: 0.10 × 10 to 0.30 × 10⁵ ppm, preferably 0.10 × 10² to 0.30 × 10⁴ ppm.
(20) cyclo-histidyl-phenylalanine or a salt thereof, content: 0.20 × 10 to 0.40 × 10⁵ ppm, preferably 0.20 × 10² to 0.40 × 10⁴ ppm.
(21) cyclo-aspartyl-leucine or a salt thereof, content: 0.40 × 10 to 0.80 × 10⁵ ppm, preferably 0.40 × 10² to 0.80 × 10⁴ ppm.
(22) cyclo-isoleucyl-histidine or a salt thereof, content: 0.70 × 10 to 1.20 × 10⁵ ppm, preferably 0.70 × 10² to 1.20 × 10⁴ ppm.
(23) cyclo-seryl-leucine or a salt thereof, content: 0.70 × 10 to 1.10 × 10⁵ ppm, preferably 0.70 × 10² to 1.10 × 10⁴ ppm.
(24) cyclo-isoleucyl-aspartic acid or a salt thereof, content: 0.70 × 10 to 1.10 × 10⁵ ppm, preferably 0.70 × 10² to 1.10 × 10⁴ ppm.
(25) cyclo-seryl-cysteine or a salt thereof, content: 0.40 × 10 to 0.70 × 10⁵ ppm, preferably 0.40 × 10² to 0.70 × 10⁴ ppm.
(26) cyclo-phenylalanyl-tryptophan or a salt thereof, content: 0.07 × 10 to 0.20 × 10⁵ ppm, preferably 0.07 × 10² to 0.20 × 10⁴ ppm.
(27) cyclo-alanyl-leucine or a salt thereof, content: 1.40 × 10 to 2.30 × 10⁵ ppm, preferably 1.40 × 10² to 2.30 × 10⁴ ppm.
(28) cyclo-glutaminyl-histidine or a salt thereof, content: 0.40 × 10 to 0.70 × 10⁵ ppm, preferably 0.40 × 10² to 0.70 × 10⁴ ppm.
(29) cyclo-arginyl-valine or a salt thereof, content: 0.60 × 10 to 1.00 × 10⁵ ppm, preferably 0.60 × 10² to 1.00 × 10⁴ ppm.
(30) cyclo-glutamyl-leucine or a salt thereof, content: 0.70 × 10 to 1.10 × 10⁵ ppm, preferably 0.70 × 10² to 1.10 × 10⁴ ppm.
(31) cyclo-leucyl-tryptophan or a salt thereof, content: 0.10 × 10 to 0.30 × 10⁵ ppm, preferably 0.10 × 10² to 0.30 × 10⁴ ppm.
(32) cyclo-tryptophanyl-tryptophan or a salt thereof, content: 0.04 × 10 to 0.07 × 10⁵ ppm, preferably 0.04 × 10² to 0.07 × 10⁴ ppm.
(33) cyclo-L-alanyl-proline or a salt thereof, content: 0.40 × 10 to 0.70 × 10⁵ ppm, preferably 0.40 × 10² to 0.70 × 10⁴ ppm.
(34) cyclo-methionyl-arginine or a salt thereof, content: 0.20 × 10 to 0.50 × 10⁵ ppm, preferably 0.20 × 10² to 0.50 × 10⁴ ppm.
(35) cyclo-lysyl-phenylalanine or a salt thereof, content: 1.00 × 10 to 1.60 × 10⁵ ppm, preferably 1.00 × 10² to 1.60 × 10⁴ ppm.
(36) cyclo-phenylalanyl-phenylalanine or a salt thereof, content: 0.30 × 10 to 0.60 × 10⁵ ppm, preferably 0.30 × 10² to 0.60 × 10⁴ ppm.
(37) cyclo-tryptophanyl-tyrosine or a salt thereof, content: 0.10 × 10 to 0.20 × 10⁵ ppm, preferably 0.10 × 10² to 0.20 × 10⁴ ppm.
(38) cyclo-asparaginyl-valine or a salt thereof, content: 0.30 × 10 to 0.50 × 10⁵ ppm, preferably 0.30 × 10² to 0.50 × 10⁴ ppm.
(39) cyclo-glutaminyl-isoleucine or a salt thereof, content: 0.50 × 10 to 0.80 × 10⁵ ppm, preferably 0.50 × 10² to 0.80 × 10⁴ ppm.
(40) cyclo-alanyl-serine or a salt thereof, content: 0.40 × 10 to 0.80 × 10⁵ ppm, preferably 0.40 × 10² to 0.80 × 10⁴ ppm.
(41) cyclo-methionyl-histidine or a salt thereof, content: 0.10 × 10 to 0.30 × 10⁵ ppm, preferably 0.10 × 10² to 0.30 × 10⁴ ppm.
(42) cyclo-methionyl-proline or a salt thereof, content: 0.10 × 10 to 0.30 × 10⁵ ppm, preferably 0.10 × 10² to 0.30 × 10⁴ ppm.
(43) cyclo-arginyl-leucine or a salt thereof, content: 1.50 × 10 to 2.30 × 10⁵ ppm, preferably 1.50 × 10² to 2.30 × 10⁴ ppm.
(44) cyclo-methionyl-glutamic acid or a salt thereof, content: 0.10 × 10 to 0.30 × 10⁵ ppm, preferably 0.10 × 10² to 0.30 × 10⁴ ppm.
(45) cyclo-methionyl-alanine or a salt thereof, content: 0.20 × 10 to 0.50 × 10⁵ ppm, preferably 0.20 × 10² to 0.50 × 10⁴ ppm.
(46) cyclo-isoleucyl-glutamic acid or a salt thereof: 0.50 × 10 to 0.90 × 10⁵ ppm, preferably 0.50 × 10² to 0.90 × 10⁴ ppm.
(47) cyclo-isoleucyl-serine or a salt thereof, content: 0.50 x 10 to 0.90 × 10⁵ ppm, preferably 0.50 × 10² to 0.90 × 10⁴ ppm.
(48) cyclo-valyl-serine or a salt thereof, content: 0.50 × 10 to 0.90 × 10⁵ ppm, preferably 0.50 × 10² to 0.90 × 10⁴ ppm.
(49) cyclo-methionyl-glycine or a salt thereof, content: 0.40 × 10 to 0.70 × 10⁵ ppm, preferably 0.40 × 10² to 0.70 × 10⁴ ppm.
(50) cyclo-valyl-threonine or a salt thereof, content: 0.30 × 10 to 0.60 × 10⁵ ppm, preferably 0.30 × 10² to 0.60 × 10⁴ ppm.
(51) cyclo-valyl-aspartic acid or a salt thereof, content: 0.50 × 10 to 0.90 × 10⁵ ppm, preferably 0.50 × 10² to 0.90 × 10⁴ ppm.
(52) cyclo-glycyl-proline or a salt thereof, content: 0.30 × 10 to 0.50 × 10⁵ ppm, preferably 0.30 × 10² to 0.50 × 10⁴ ppm.
(53) cyclo-leucyl-proline or a salt thereof, content: 0.50 × 10 to 0.90 × 10⁵ ppm, preferably 0.50 × 10² to 0.90 × 10⁴ ppm.
(54) cyclo-glutaminyl-glycine or a salt thereof, content: 0.05 × 10 to 0.09 × 10⁵ ppm, preferably 0.05 × 10² to 0.09 × 10⁴ ppm.
(55) cyclo-tryptophanyl-lysine or a salt thereof, content: 0.20 × 10 to 0.40 × 10⁵ ppm, preferably 0.20 × 10² to 0.40 × 10⁴ ppm.
(56) cyclo-glutaminyl-phenylalanine or a salt thereof, content: 0.30 × 10 to 0.60 × 10⁵ ppm, preferably 0.30 × 10² to 0.60 × 10⁴ ppm.
(57) cyclo-lysyl-glycine or a salt thereof, content: 1.00 × 10 to 1.60 × 10⁵ ppm, preferably 1.00 × 10² to 1.60 × 10⁴ ppm.
(58) cyclo-seryl-lysine or a salt thereof, content: 1.60 × 10 to 2.60 × 10⁵ ppm, preferably 1.60 × 10² to 2.60 × 10⁴ ppm.
(59) cyclo-valyl-lysine or a salt thereof, content: 0.90 × 10 to 1.50 × 10⁵ ppm, preferably 0.90 × 10² to 1.50 × 10⁴ ppm.
(60) cyclo-asparaginyl-lysine or a salt thereof, content: 0.60 × 10 to 1.10 × 10⁵ ppm, preferably 0.60 × 10² to 1.10 × 10⁴ ppm.
(61) cyclo-histidyl-histidine or a salt thereof, content: 0.40 × 10 to 0.70 × 10⁵ ppm, preferably 0.40 × 10² to 0.70 × 10⁴ ppm.
(62) cyclo-threonyl-histidine or a salt thereof, content: 0.40 × 10 to 0.70 × 10⁵ ppm, preferably 0.40 × 10² to 0.70 × 10⁴ ppm.
(63) cyclo-aspartyl-histidine or a salt thereof, content: 0.40 × 10 to 0.70 × 10⁵ ppm, preferably 0.40 × 10² to 0.70 × 10⁴ ppm.
(64) cyclo-asparaginyl-histidine or a salt thereof, content: 0.10 × 10 to 0.30 × 10⁵ ppm, preferably 0.10 × 10² to 0.30 × 10⁴ ppm.
(65) cyclo-arginyl-serine or a salt thereof, content: 0.40 × 10 to 0.70 × 10⁵ ppm, preferably 0.40 × 10² to 0.70 × 10⁴ ppm.
(66) cyclo-asparaginyl-methionine or a salt thereof, content: 0.40 × 10 to 0.70 × 10⁵ ppm, preferably 0.40 × 10² to 0.70 × 10⁴ ppm.
(67) cyclo-glutaminyl-methionine or a salt thereof, content: 0.10 × 10 to 0.20 × 10⁵ ppm, preferably 0.10 × 10² to 0.20 × 10⁴ ppm.
(68) cyclo-tryptophanyl-arginine or a salt thereof, content: 0.10 × 10 to 0.30 × 10⁵ ppm, preferably 0.10 × 10² to 0.30 × 10⁴ ppm.
(69) cyclo-asparaginyl-arginine or a salt thereof, content: 0.40 × 10 to 0.70 × 10⁵ ppm, preferably 0.40 × 10² to 0.70 × 10⁴ ppm.
(70) cyclo-asparaginyl-proline or a salt thereof, content: 0.05 × 10 to 0.09 × 10⁵ ppm, preferably 0.05 × 10² to 0.09 × 10⁴ ppm.
(71) cyclo-arginyl-arginine or a salt thereof, content: 0.40 × 10 to 0.70 × 10⁵ ppm, preferably 0.40 × 10² to 0.70 × 10⁴ ppm.

The carbohydrate metabolism-ameliorating agent according to the present invention is administered in a suitable administration method in accordance with its form. The administration method is not particularly limited and may be any method which allows the cyclic dipeptide or salt thereof according to the present invention to be transferred into the circulating blood. For example, the carbohydrate metabolism-ameliorating agent may be administered in internal administration, external administration, intradermal administration, etc., and may be administered in a suitable administration method, for example, in an external preparation such as a suppository in the case of external administration.

The dose of the carbohydrate metabolism-ameliorating agent or GLP-1 secretion accelerator according to the present invention is not constant, and is set appropriately in accordance with its form, administration method, intended use, and the age, body weight, and symptoms of a patient or patient animal as a subject for administration of the carbohydrate metabolism-ameliorating agent. In the present invention, for example, the effective amount of ingestion of the cyclic dipeptide or salt thereof according to the present invention for a human is, in the case of a human with a body weight of 50 kg, preferably 0.2 mg or more, more preferably 2 mg or more, further preferably 20 mg or more and preferably 10 g or less, more preferably 5 g or less, further preferably 2 g or less per day. Administration may be performed in a single administration or multiple administrations in a day, within a desired dose range. Any duration may be used for the administration. Here, the effective amount of ingestion of the cyclic dipeptide or salt thereof according to the present invention for a human refers to the total amount of ingestion of the cyclic dipeptide or salt thereof which provides an effective action on a human, and the type of the cyclic dipeptide is not particularly limited.

Throughout the specification, the subject to ingest the carbohydrate metabolism-ameliorating agent or a GLP-1 secretion accelerator according to the present invention is preferably a human, but may be a domestic animal such as a cattle, a horse, and a goat, a pet animal such as a dog, a cat, and a rabbit, or a laboratory animal such as a mouse, a rat, a guinea pig, and a monkey.

The present invention further provide a method for ameliorating carbohydrate metabolism including administering a therapeutically effective amount of the cyclic dipeptide or salt thereof according to the present invention or a composition containing the cyclic dipeptide or salt thereof according to the present invention to an individual in need of amelioration of carbohydrate metabolism. Here, an individual in need of amelioration of carbohydrate metabolism refers to the above subject for administration of the carbohydrate metabolism-ameliorating agent according to the present invention.

Throughout the specification, a therapeutically effective amount refers to an amount at which carbohydrate metabolism in the individual administered with the cyclic dipeptide or salt thereof according to the present invention is ameliorated compared to the case of an individual without administration. A specific effective amount is not constant, and is set appropriately in accordance with form of administration, an administration method, intended use, and the age, body weight, and symptoms of an individual.

In the therapeutic method according to the present invention, the cyclic dipeptide or salt thereof according to the present invention may be administered as it is, or may be administered as a composition containing the cyclic dipeptide or salt thereof according to the present invention. The administration method is not limited, and administration may be carried out orally, for example.

The therapeutic method according to the present invention enables amelioration of carbohydrate metabolism without any side effect.

### EXAMPLES

The present invention will now be described with reference to Examples, but is not limited to the following Examples.

### <Reagents>

Cyclic dipeptides were synthesized by KNC Laboratories Co., Ltd. Used were a Poly-L-Lysine 96-well plate manufactured by BD Biosciences; PBS(+), an antibiotic, a Dulbecco's Modified Eagle's Medium (DMEM), glucose, and sodium carboxymethylcellulose (CMC-Na) manufactured by NACALAI TESQUE, INC.; TPA manufactured by Cell Signaling Technology, Inc.; an active GLP-1 ELISA kit manufactured by Merck Millipore Corporation; Fetal bovine serum (FBS) manufactured by Sigma-Aldrich Co., LLC.; Sitagliptin phosphate manufactured by Santa Cruz Biotechnology, Inc.; a Glutest Neo Super and a Glutest Neo Sensor manufactured by SANWA KAGAKU KENKYUSHO CO., LTD.; soybean peptides (HINUTE AM) manufactured by Fuji Oil Co., Ltd.; and NCI-H716 cells donated by ATCC.

### <Statistical analysis>

In the Test Examples below, data were represented as average value ± standard error. In Test Examples 1, 2, and 3, a Student's t-test was used for a statistical test, and in other Test Examples, one-way ANOVA was carried out for dispersion analysis followed by a multiple comparison test by using a Dunnet's test. "*" and "#" in the results indicate the presence of a significant difference at p < 0.05 and the presence of a significant difference at p < 0.1, respectively. All of these analyses were carried out by using an SPSS for Windows release 17.0 (manufactured by SPSS Inc.).

Test Example 1 (Study of in vitro GLP-1 secretion-accelerating action by using cyclic dipeptide)

Among the cyclic dipeptides, 92 highly water-soluble cyclic dipeptides were used in the following experiment. In each well of a Poly-L-Lysine 96-well plate, NCI-H716 cells suspended in 100 µL of a DMEM (with 10% FBS, 2 mM glutamine, and 1% antibiotics added in advance) were seeded at 0.5 × 10⁵ cells/well, and cultured in a CO₂ incubator (manufactured by ESPEC CORP.) for 48 hours. After washing with PBS(+), 100 µL of a PBS(+) solution with each cyclic dipeptide at a final concentration of 10 mM and 10 µM of Sitagliptin phosphate added therein was added to the cells. After 1 hour, the resultant solution was recovered, and the amount of active GLP-1 in the solution was measured by using the ELISA kit. In the analysis, the amount of active GLP-1 for a group with no cyclic dipeptide added was defined as 100, and relative values thereto were used.

Tables 1 to 5 show the results. In each Table, cyclic dipeptides for which an in vitro active GLP-1 secretion-accelerating activity was found and was not found are listed.

**[Table 1]**

| Experiment 1 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 23 | | |
| 1 | Cyclo(Ser-Ser) | 124 | 17 | 0.221 | No |
| 3 | Cyclo(Ala-Ala) | 95 | 7 | 0.418 | No |
| 5 | Cyclo(D-Ala-Pro) | 113 | 19 | 0.347 | No |
| 6 | Cyclo(Pro-Pro) | 87 | 20 | 0.348 | No |
| 7 | Cyclo(Phe-Ser) | 106 | 24 | 0.435 | No |
| 10 | Cyclo(Val-Pro) | 95 | 21 | 0.440 | No |
| 18 | Cyclo(Met-Pro) | 338 | 45 | 0.005 | Yes |
| 19 | Cyclo(Leu-Pro) | 173 | 16 | 0.031 | Yes |
| 25 | Cyclo(Ala-Ser) | 257 | 56 | 0.031 | Yes |
| 31 | Cyclo(Pro-Thr) | 204 | 32 | 0.029 | Yes |
| 34 | Cyclo(Ala-His) | 211 | 56 | 0.070 | No |
| 37 | Cyclo(His-Pro) | 231 | 88 | 0.113 | No |
| 40 | Cyclo(Lys-Lys) | 164 | 73 | 0.225 | No |
| 43 | Cyclo(Arg-Leu) | 226 | 24 | 0.010 | Yes |
| 45 | Cyclo(Glu-Pro) | 145 | 5 | 0.066 | No |

**[Table 2]**

| Experiment 2 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 6 | | |
| 46 | Cyclo(Ser-Pro) | 139 | 36 | 0.173 | No |
| 47 | Cyclo(Ile-Pro) | 79 | 16 | 0.146 | No |
| 48 | Cyclo(Asp-Pro) | 199 | 15 | 0.002 | Yes |
| 57 | Cyclo(Trp-Pro) | 81 | 11 | 0.111 | No |
| 58 | Cyclo(Lys-Pro) | 91 | 14 | 0.288 | No |
| 59 | Cyclo(Trp-Lys) | 239 | 48 | 0.022 | Yes |
| 60 | Cyclo(Trp-His) | 260 | 32 | 0.004 | Yes |
| 63 | Cyclo(Lys-Phe) | 220 | 20 | 0.002 | Yes |
| 65 | Cyclo(Gln-Phe) | 227 | 41 | 0.019 | Yes |
| 68 | Cyclo(Leu-Lys) | 208 | 51 | 0.053 | No |
| 69 | Cyclo(Ala-Lys) | 161 | 56 | 0.168 | No |
| 73 | Cyclo(Hyp-Gly) | 121 | 18 | 0.171 | No |
| 74 | Cyclo(Hyp-Pro) | 115 | 30 | 0.325 | No |
| 78 | Cyclo(Trp-Ser) | 119 | 32 | 0.301 | No |

**[Table 3]**

| Experiment 3 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 7 | | |
| 96 | Cyclo(Lys-Gly) | 133 | 13 | 0.046 | Yes |
| 97 | Cyclo(Glu-Lys) | 66 | 25 | 0.131 | No |
| 98 | Cyclo(Ser-Lvs) | 71 | 7 | 0.023 | Yes |
| 99 | Cyclo(Val-Lys) | 532 | 21 | 0.000 | Yes |
| 100 | Cyclo(Asp-Lys) | 116 | 42 | 0.360 | No |
| 101 | Cyclo(Asn-Lys) | 250 | 64 | 0.040 | Yes |
| 102 | Cyclo(Gln-Lys) | 112 | 12 | 0.228 | No |
| 107 | Cyclo(His-His) | 182 | 14 | 0.003 | Yes |
| 109 | Cyclo(Thr-His) | 290 | 7 | 0.000 | Yes |
| 110 | Cyclo(Asp-His) | 340 | 7 | 0.000 | Yes |
| 111 | Cyclo(Asn-His) | 341 | 33 | 0.001 | Yes |
| 112 | Cyclo(Arg-His) | 106 | 47 | 0.450 | No |
| 113 | Cyclo(Glu-Ala) | 157 | 36 | 0.100 | No |
| 114 | Cyclo(Thr-Ala) | 89 | 18 | 0.300 | No |
| 116 | Cyclo(Thr-Gly) | 146 | 36 | 0.140 | No |
| 117 | Cyclo(Arg-Gly) | 98 | 16 | 0.466 | No |
| 121 | Cyclo(Gly-Ala) | 153 | 61 | 0.221 | No |
| 124 | Cyclo(Asn-Ala) | 116 | 9 | 0.122 | No |
| 126 | Cyclo(Gln-Gly) | 210 | 48 | 0.044 | Yes |
| 129 | Cyclo(Asn-Glu) | 118 | 9 | 0.109 | No |
| 130 | Cyclo(Gln-Glu) | 120 | 8 | 0.078 | No |
| 136 | Cyclo(Gln-Ser) | 251 | 79 | 0.066 | No |
| 143 | Cyclo(Thr-Lys) | 105 | 21 | 0.413 | No |
| 153 | Cyclo(Asp-Ala) | 94 | 14 | 0.362 | No |
| 154 | Cyclo(Ser-Gly) | 116 | 2 | 0.053 | No |
| 157 | Cyclo(Thr-Glu) | 139 | 25 | 0.108 | No |
| 159 | Cyclo(Arg-Ser) | 135 | 14 | 0.048 | Yes |
| 164 | Cyclo(Thr-Thr) | 162 | 23 | 0.032 | Yes |
| 165 | Cyclo(Asp-Thr) | 140 | 28 | 0.121 | No |
| 166 | Cyclo(Asn-Thr) | 149 | 52 | 0.200 | No |
| 167 | Cyclo(Gln-Thr) | 156 | 61 | 0.204 | No |
| 168 | Cyclo(Arg-Thr) | 142 | 58 | 0.255 | No |
| 170 | Cyclo(Asn-Asp) | 125 | 13 | 0.089 | No |
| 171 | Cyclo(Gln-Asp) | 120 | 21 | 0.214 | No |

**[Table 4]**

| Experiment 4 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 27 | | |
| 172 | Cyclo(Met-Asp) | 141 | 40 | 0.220 | No |
| 173 | Cyclo(Asn-Asn) | 255 | 19 | 0.005 | Yes |
| 174 | Cyclo(Gln-Asn) | 305 | 64 | 0.021 | Yes |
| 175 | Cyclo(Asn-Met) | 218 | 53 | 0.059 | Yes |
| 176 | Cyclo(Gln-Gln) | 111 | 22 | 0.379 | No |
| 182 | Cyclo(Gln-Ile) | 445 | 51 | 0.002 | Yes |
| 186 | Cyclo(Gln-Met) | 191 | 7 | 0.015 | Yes |
| 187 | Cyclo(L-Ala-Pro) | 1186 | 171 | 0.002 | Yes |
| 188 | Cyclo(Gln-Pro) | 132 | 5 | 0.153 | No |
| 189 | Cyclo(Trp-Arg) | 314 | 39 | 0.005 | Yes |
| 191 | Cyclo(Gln-His) | 743 | 102 | 0.002 | Yes |
| 192 | Cyclo(Ser-Glu) | 993 | 77 | 0.000 | Yes |
| 193 | Cyclo(Asp-Ser) | 509 | 40 | 0.001 | Yes |
| 194 | Cyclo(Arg-Val) | 498 | 45 | 0.001 | Yes |
| 197 | Cyclo(Asn-Arg) | 359 | 73 | 0.015 | Yes |
| 199 | Cyclo(Met-Arg) | 652 | 73 | 0.001 | Yes |
| 201 | Cyclo(Asn-Pro) | 430 | 73 | 0.007 | Yes |
| 207 | Cyclo(Arg-Asp) | 1255 | 61 | 0.000 | Yes |
| 209 | Cyclo(Arg-Pro) | 441 | 29 | 0.000 | Yes |
| 212 | Cyclo(Arg-Arg) | 1050 | 58 | 0.000 | Yes |
| 215 | Cyclo(Asn-Gly) | 611 | 49 | 0.000 | Yes |

**[Table 5]**

| Experiment 5 | | | | | |
|---|---|---|---|---|---|
| No. | Compound | Amount of active GLP-1 secreted (relative value to control group as 100) | | p value (vs control group) | Significant difference (p<0.05) |
| | | Average value | Standard error | | |
| Control group | None | 100 | 6 | 0.086 | No |
| 2 | Cyclo(Gly-Gly) | 151 | 32 | 0.095 | No |
| 4 | Cyclo(Gly-Pro) | 157 | 125 | 0.044 | Yes |
| 21 | Cyclo(Gly-Leu) | 131 | 24 | 0.141 | No |
| 23 | Cyclo(Ala-Gln) | 99 | 26 | 0.480 | No |
| 24 | Cyclo(Glu-Gly) | 89 | 15 | 0.267 | No |
| 27 | Cyclo(Gly-His) | 82 | 20 | 0.216 | No |
| 32 | Cyclo(Asp-Gly) | 12 | 3 | 0.000 | Yes |
| 36 | Cyclo(Gln-Gly) | 75 | 15 | 0.099 | No |

### Test Example 2 (Study of in vivo plasma GLP-1 level-increasing action by using cyclic dipeptide)

Among the cyclic dipeptides, cyclic dipeptides for which an in vitro GLP-1 secretion-accelerating action was found and highly lipid-soluble cyclic dipeptides were used in the following experiment. Seven-week-old male C57/BL6J mice were purchased from CLEA Japan, Inc. for use, and they were subjected to an experiment after 1 week of a habituation period. Each animal was grown in an animal room with an air conditioning system (temperature: 23.5 ± 1.0°C, humidity: 55 ± 10RH%, air ventilation: 12 to 15 times/hour, illumination: from 7:00 to 19:00/day). During the habituation period, the mice had a free access to a commercially available feed (CE-2, manufactured by CLEA Japan, Inc.) and tap water.

After the habituation period, the mice were subjected to 5.5 hours of food deprivation and 2 hours of water deprivation. Thereafter, a cyclic dipeptide dissolved or suspended in a 0.5% CMC-Na aqueous solution in 10 mg/kg was orally administered to each mouse forcibly. After 30 minutes, the blood was collected from the abdominal vena cava under anesthesia, and 1 µL of heparin sodium and 1 µL of 10 mM Sitagliptin phosphate were added to each collected blood, which was then subjected to centrifugation at 8000 rpm for 10 minutes to recover the plasma, and the plasma active GLP-1 level was measured by using the ELISA kit. In the analysis, the active GLP-1 level for a group administered with 0.5% CMC-Na aqueous solution was defined as 100, and relative values thereto were used.

Tables 6 to 15 show the results. In each Table, cyclic dipeptides for which an in vivo plasma active GLP-1 level-increasing action was found and was not found are listed.

**[Table 6]**

| Experiment 1 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 10 | | |
| 18 | Cyclo(Met-Pro) | 138 | 10 | 0.024 | Yes |
| 25 | Cyclo(Ala-Ser) | 158 | 10 | 0.007 | Yes |
| 31 | Cyclo(Pro-Thr) | 129 | 10 | 0.051 | No |
| 34 | Cyclo(Ala-His) | 100 | 67 | 0.500 | No |
| 37 | Cyclo(His-Pro) | 62 | 0 | 0.008 | Yes |
| 43 | Cyclo(Arg-Leu) | 138 | 10 | 0.024 | Yes |
| 48 | Cyclo(Asp-Pro) | 110 | 10 | 0.259 | No |
| 54 | Cyclo(Trp-Lys) | 119 | 0 | 0.058 | No |
| 60 | Cyclo(Trp-His) | 129 | 10 | 0.051 | No |
| 63 | Cyclo(Lvs-Phe) | 206 | 44 | 0.039 | Yes |

**[Table 7]**

| Experiment 2 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 10 | | |
| 182 | Cyclo(Gln-Ile) | 188 | 10 | 0.002 | Yes |
| 187 | Cyclo(L-Ala-Pro) | 217 | 10 | 0.001 | Yes |
| 191 | Cyclo(Gln-His) | 247 | 10 | 0.000 | Yes |
| 192 | Cyclo(Ser-Glu) | 286 | 17 | 0.000 | Yes |
| 193 | Cyclo(Asp-Ser) | 266 | 10 | 0.000 | Yes |
| 194 | Cyclo(Arg-Val) | 237 | 20 | 0.002 | Yes |
| 199 | Cyclo(Met-Arg) | 217 | 10 | 0.001 | Yes |
| 207 | Cyclo(Arg-Asp) | 266 | 26 | 0.002 | Yes |
| 209 | Cyclo(Arg-Pro) | 315 | 29 | 0.001 | Yes |
| 215 | Cyclo(Asn-Gly) | 305 | 10 | 0.000 | Yes |

**[Table 8]**

| Experiment 3 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 17 | | |
| 17 | Cyclo(Phe-Phe) | 203 | 9 | 0.003 | Yes |
| 22 | Cyclo(Trp-Tyr) | 203 | 9 | 0.003 | Yes |
| 26 | Cyclo(Gly-Trp) | 264 | 17 | 0.001 | Yes |
| 28 | Cyclo(Phe-Trp) | 255 | 17 | 0.002 | Yes |
| 38 | Cyclo(His-Phe) | 264 | 9 | 0.001 | Yes |
| 39 | Cyclo(Leu-Trp) | 229 | 9 | 0.001 | Yes |
| 44 | Cyclo(Ala-Leu) | 255 | 9 | 0.001 | Yes |
| 61 | Cyclo(Trp-Val) | 117 | 0 | 0.187 | No |
| 62 | Cyclo(Trp-Ile) | 178 | 71 | 0.173 | No |
| 75 | Cyclo(Trp-Trp) | 229 | 34 | 0.014 | Yes |

**[Table 9]**

| Experiment 4 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 10 | | |
| 8 | Cyclo(Gly-Phe) | 85 | 5 | 0.125 | No |
| 11 | Cyclo(Val-Val) | 100 | 13 | 0.500 | No |
| 12 | Cyclo(Met-Met) | 110 | 10 | 0.259 | No |
| 13 | Cyclo(Phe-Pro) | 120 | 9 | 0.103 | No |
| 14 | Cyclo(Ile-Ile) | 105 | 17 | 0.407 | No |
| 15 | Cyclo(Leu-Leu) | 95 | 10 | 0.371 | No |
| 16 | Cyclo(Leu-Phe) | 105 | 15 | 0.398 | No |
| 29 | Cyclo(Ser-Tyr) | 95 | 5 | 0.339 | No |
| 30 | Cyclo(Pro-Tyr) | 95 | 10 | 0.371 | No |
| 33 | Cyclo(Asp-Phe) | 90 | 9 | 0.246 | No |

**[Table 10]**

| Experiment 5 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 6 | | |
| 42 | Cyclo(Tyr-Gly) | 97 | 3 | 0.322 | No |
| 49 | Cyclo(Tyr-Tyr) | 100 | 0 | 0.500 | No |
| 52 | Cyclo(Ala-Phe) | 93 | 3 | 0.187 | No |
| 53 | Cyclo(Glu-Phe) | 93 | 3 | 0,187 | No |
| 54 | Cyclo(Val-Phe) | 93 | 9 | 0.281 | No |
| 55 | Cyclo(Ile-Phe) | 90 | 6 | 0.144 | No |
| 56 | Cyclo(Thr-Phe) | 87 | 3 | 0.058 | No |
| 64 | Cyclo(Asn-Phe) | 93 | 3 | 0.187 | No |
| 67 | Cyclo(Met-Phe) | 93 | 9 | 0.281 | No |
| 70 | Cyclo(Met-Lys) | 93 | 3 | 0.187 | No |

**[Table 11]**

| Experiment 6 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 0 | | |
| 71 | Cyclo(Ile-Leu) | 79 | 10 | 0.058 | No |
| 72 | Cyclo(Met-Leu) | 90 | 10 | 0.187 | No |
| 76 | Cyclo(Trp-Ala) | 110 | 10 | 0.187 | No |
| 77 | Cyclo(Trp-Glu) | 110 | 21 | 0.322 | No |
| 79 | Cyclo(Trp-Thr) | 100 | 18 | 0.500 | No |
| 81 | Cyclo(Trp-Asn) | 100 | 0 | 1.000 | No |
| 82 | Cyclo(Trp-Gln) | 90 | 10 | 0.187 | No |
| 83 | Cyclo(Trp-Met) | 110 | 10 | 0.187 | No |
| 85 | Cyclo(His-Tyr) | 90 | 10 | 0.187 | No |
| 86 | Cyclo(Ala-Tyr) | 90 | 21 | 0.322 | No |

**[Table 12]**

| Experiment 7 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 81 | | |
| 87 | Cyclo(Glu-Tyr) | 141 | 71 | 0.362 | No |
| 88 | Cyclo(Val-Tyr) | 385 | 71 | 0.029 | Yes |
| 89 | Cyclo(Ile-Tyr) | 222 | 108 | 0.208 | No |
| 90 | Cyclo(Thr-Tyr) | 466 | 108 | 0.027 | Yes |
| 91 | Cyclo(Asp-Tyr) | 181 | 81 | 0.259 | No |
| 92 | Cyclo(Asn-Tyr) | 181 | 41 | 0.211 | No |
| 93 | Cyclo(Gln-Tyr) | 344 | 41 | 0.028 | Yes |
| 94 | Cyclo(Arg-Tyr) | 222 | 41 | 0.125 | No |

**[Table 13]**

| Experiment 8 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | | 100 | 10 | | |
| 103 | Cyclo(His-Leu) | 94 | 12 | 0.362 | No |
| 105 | Cyclo(Thr-Leu) | 94 | 6 | 0.322 | No |
| 106 | Cyclo(Asn-Leu) | 94 | 6 | 0.322 | No |
| 108 | Cyclo(Val-His) | 118 | 18 | 0.218 | No |
| 118 | Cyclo(Val-Glu) | 112 | 16 | 0.281 | No |
| 119 | Cyclo(Met-Ile) | 106 | 6 | 0.322 | No |
| 120 | Cyclo(Met-His) | 154 | 18 | 0.030 | Yes |
| 122 | Cyclo(Val-Ala) | 94 | 6 | 0.322 | No |

**[Table 14]**

| Experiment 9 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | None | 100 | 7 | | |
| 123 | Cyclo(Ile-Ala) | 120 | 11 | 0.095 | No |
| 125 | Cyclo(Met-Ala) | 133 | 11 | 0.032 | Yes |
| 127 | Cyclo(Met-Gly) | 124 | 0 | 0.013 | Yes |
| 128 | Cyclo(Ile-Glu) | 133 | 4 | 0.008 | Yes |
| 131 | Cyclo(Met-Glu) | 137 | 0 | 0.003 | Yes |
| 132 | Cyclo(Val-Ser) | 129 | 4 | 0.012 | Yes |
| 133 | Cyclo(Ile-Ser) | 133 | 8 | 0.020 | Yes |
| 138 | Cyclo(Ile-Val) | 96 | 4 | 0.322 | No |
| 139 | Cyclo(Val-Thr) | 120 | 4 | 0.033 | Yes |
| 140 | Cyclo(Val-Asp) | 120 | 4 | 0.033 | Yes |

**[Table 15]**

| Experiment 10 | | | | | |
|---|---|---|---|---|---|
| No. | Compound | Plasma active GLP-1 level (relative value to control group as 100) | | p value (vs control group) | Significant difference (p<0.05) |
| | | Average value | Standard error | | |
| Control group | | 100 | 23 | | |
| 141 | Cyclo(Asn-Val) | 191 | 23 | 0.024 | Yes |
| 145 | Cyclo(Glu-Leu) | 236 | 23 | 0.007 | Yes |
| 146 | Cyclo(Asp-Leu) | 259 | 23 | 0.004 | Yes |
| 148 | Cyclo(Ile-His) | 259 | 23 | 0.004 | Yes |
| 150 | Cyclo(Ile-Lys) | 282 | 0 | 0.001 | Yes |
| 151 | Cyclo(Ser-Leu) | 259 | 23 | 0.004 | Yes |
| 152 | Cyclo(Gln-Leu) | 282 | 0 | 0.001 | Yes |
| 155 | Cyclo(Val-Gly) | 55 | 91 | 0.326 | No |
| 156 | Cyclo(Ile-Gly) | 145 | 39 | 0.187 | No |
| 160 | Cyclo(Met-Val) | 304 | 60 | 0.017 | Yes |
| 161 | Cyclo(Ile-Thr) | 282 | 0 | 0.001 | Yes |
| 162 | Cyclo(Ile-Asp) | 259 | 45 | 0.018 | Yes |
| 163 | Cyclo(Asn-Ile) | 327 | 23 | 0.001 | Yes |
| 164 | Cyclo(Thr-Thr) | 282 | 0 | 0.001 | Yes |
| 169 | Cyclo(Met-Thr) | 282 | 0 | 0.001 | Yes |
| 173 | Cyclo(Asn-Asn) | 123 | 45 | 0.339 | No |
| 174 | Cyclo(Gln-Asn) | 168 | 23 | 0.051 | No |
| 177 | Cyclo(Ala-Cys) | 282 | 39 | 0.008 | Yes |
| 178 | Cyclo(Gly-Cys) | 282 | 0 | 0.001 | Yes |
| 179 | Cyclo(Glu-Cys) | 304 | 45 | 0.008 | Yes |
| 180 | Cyclo(Ser-Cys) | 259 | 23 | 0.004 | Yes |

### Test Example 3 (Study of in vivo plasma GLP-1 level-increasing action by using heat-treated soybean peptide)

A heat-treated soybean peptide was used in the following experiment. Seven-week-old male C57/BL6J mice were purchased from CLEA Japan, Inc. for use, and they were subjected to an experiment after 1 week of a habituation period. Each animal was grown in an animal room with an air conditioning system (temperature: 23.5 ± 1.0°C, humidity: 55 ± 10RH%, air ventilation: 12 to 15 times/hour, illumination: from 7:00 to 19:00/day). During the habituation period, the mice had a free access to a commercially available feed (CE-2, manufactured by CLEA Japan, Inc.) and tap water.

After the habituation period, the mice were subjected to 5.5 hours of food deprivation and 2 hours of water deprivation. Thereafter, a heat-treated soybean peptide dissolved in distilled water in 1 g/kg was orally administered to each mouse forcibly. After 15 minutes, 30 minutes, and 60 minutes, the blood was collected from the abdominal vena cava under anesthesia, and 1 µL of heparin sodium and 1 µL of 10 mM Sitagliptin phosphate were added to each collected blood, which was then subjected to centrifugation at 8000 rpm for 10 minutes to recover the plasma, and the plasma active GLP-1 level was measured by using the ELISA kit.

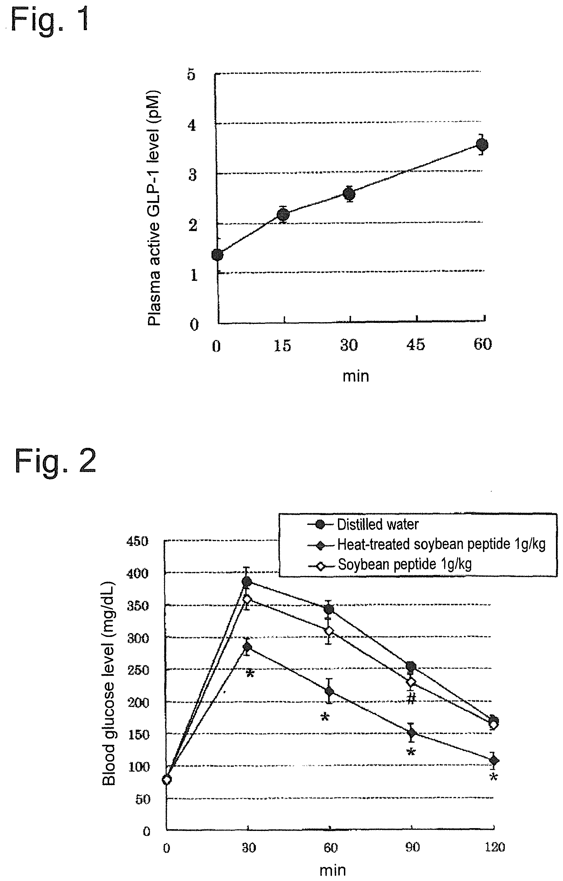

Fig. 1 shows the results.

### Test Example 4 (Study of in vivo carbohydrate metabolism-enhancing action by using heat-treated soybean peptide)

A heat-treated soybean peptide and a soybean peptide were used in the following experiment. Seven-week-old male C57/BL6J mice were purchased from CLEA Japan, Inc. for use, and they were subjected to an experiment after 1 week of a habituation period. Each animal was grown in an animal room with an air conditioning system (temperature: 23.5 ± 1.0°C, humidity: 55 ± 10RH%, air ventilation: 12 to 15 times/hour, illumination: from 7:00 to 19:00/day). During the habituation period, the mice had a free access to a commercially available feed (CE-2, manufactured by CLEA Japan, Inc.) and tap water.

After the habituation period, the mice were subjected to 5.5 hours of food deprivation and 2 hours of water deprivation. Thereafter, a heat-treated soybean peptide or a soybean peptide dissolved in distilled water in 1 g/kg was orally administered to each mouse forcibly. To a control group, the equivalent amount of distilled water was orally administered forcibly. After 30 minutes, 1 g/kg of glucose was intraperitoneally administered to all of the groups. The blood was collected from the tail vein before the intraperitoneal administration of glucose and 30 minutes, 60 minutes, 90 minutes, and 120 minutes after the intraperitoneal administration of glucose, and the blood glucose level was measured by using a Glucose Neo Super and a Glucose Neo Sensor.

Fig. 2 shows the results.

### Test Example 5 (Study of in vivo carbohydrate metabolism-enhancing action by using heat-treated tea peptide)

A heat-treated tea peptide was used in the following experiment. Seven-week-old male C57/BL6J mice were purchased from CLEA Japan, Inc. for use, and they were subjected to an experiment after 1 week of a habituation period. Each animal was grown in an animal room with an air conditioning system (temperature: 23.5 ± 1.0°C, humidity: 55 ± 10RH%, air ventilation: 12 to 15 times/hour, illumination: from 7:00 to 19:00/day). During the habituation period, the mice had a free access to a commercially available feed (CE-2, manufactured by CLEA Japan, Inc.) and tap water.

After the habituation period, the mice were subjected to 5.5 hours of food deprivation and 2 hours of water deprivation. Thereafter, a heat-treated tea peptide dissolved in distilled water in 1 g/kg was orally administered to each mouse forcibly. To a control group, the equivalent amount of distilled water was orally administered forcibly. After 30 minutes, 1 g/kg of glucose was intraperitoneally administered to all of the groups. The blood was collected from the tail vein before the intraperitoneal administration of glucose and 30 minutes, 60 minutes, 90 minutes, and 120 minutes after the intraperitoneal administration of glucose, and the blood glucose level was measured by using a Glucose Neo Super and a Glucose Neo Sensor.

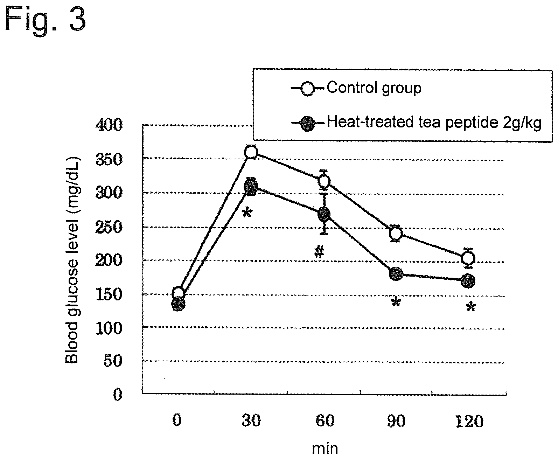

Fig. 3 shows the results.

## Claims

1. A carbohydrate metabolism-ameliorating agent comprising a cyclic dipeptide including amino acids as constituent units or a salt thereof as an active ingredient, wherein the cyclic dipeptide or salt thereof is one or two or more cyclic dipeptides selected from the group consisting of cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, cyclo-isoleucyl-lysine, cyclo-glutaminyl-leucine, cyclo-isoleucyl-threonine, cyclo-threonyl-threonine, cyclo-methionyl-threonine, cyclo-alanyl-cysteine, cyclo-glycyl-cysteine, cyclo-aspartyl-serine, cyclo-arginyl-aspartic acid, cyclo-glycyl-tryptophan, cyclo-histidyl-phenylalanine, cyclo-aspartyl-leucine, cyclo-isoleucyl-histidine, cyclo-seryl-leucine, cyclo-isoleucyl-aspartic acid, cyclo-seryl-cysteine, cyclo-phenylalanyl-tryptophan, cyclo-alanyl-leucine, cyclo-glutaminyl-histidine, cyclo-arginyl-valine, cyclo-glutamyl-leucine, cyclo-leucyl-tryptophan, cyclo-tryptophanyl-tryptophan, cyclo-L-alanyl-proline, cyclo-methionyl-arginine, cyclo-lysyl-phenylalanine, cyclo-phenylalanyl-phenylalanine, cyclo-tryptophanyl-tyrosine, cyclo-asparaginyl-valine, cyclo-glutaminyl-isoleucine, cyclo-alanyl-serine, cyclo-methionyl-histidine, cyclo-methionyl-proline, cyclo-arginyl-leucine, cyclo-methionyl-glutamic acid, cyclo-methionyl-alanine, cyclo-isoleucyl-glutamic acid, cyclo-isoleucyl-serine, cyclo-valyl-serine, cyclo-methionyl-glycine, cyclo-valyl-threonine, cyclo-valyl-aspartic acid, cyclo-glycyl-proline, cyclo-leucyl-proline, cyclo-glutaminyl-glycine, cyclo-tryptophanyl-lysine, cyclo-glutaminyl-phenylalanine, cyclo-lysyl-glycine, cyclo-seryl-lysine, cyclo-valyl-lysine, cyclo-asparaginyl-lysine, cyclo-histidyl-histidine, cyclo-threonyl-histidine, cyclo-aspartyl-histidine, cyclo-asparaginyl-histidine, cyclo-arginyl-serine, cyclo-asparaginyl-methionine, cyclo-glutaminyl-methionine, cyclo-tryptophanyl-arginine, cyclo-asparaginyl-arginine, cyclo-asparaginyl-proline, and cyclo-arginyl-arginine or salts thereof.

2. The carbohydrate metabolism-ameliorating agent according to claim 1, wherein the cyclic dipeptide or salt thereof is three or more cyclic dipeptides selected from the group defined in claim 1 or salts thereof.

3. A GLP-1 secretion accelerator comprising a cyclic dipeptide including amino acids as constituent units or a salt thereof as an active ingredient, wherein the cyclic dipeptide or salt thereof is one or two or more cyclic dipeptides selected from the group consisting of cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, cyclo-isoleucyl-lysine, cyclo-glutaminyl-leucine, cyclo-isoleucyl-threonine, cyclo-threonyl-threonine, cyclo-methionyl-threonine, cyclo-alanyl-cysteine, cyclo-glycyl-cysteine, cyclo-aspartyl-serine, cyclo-arginyl-aspartic acid, cyclo-glycyl-tryptophan, cyclo-histidyl-phenylalanine, cyclo-aspartyl-leucine, cyclo-isoleucyl-histidine, cyclo-seryl-leucine, cyclo-isoleucyl-aspartic acid, cyclo-seryl-cysteine, cyclo-phenylalanyl-tryptophan, cyclo-alanyl-leucine, cyclo-glutaminyl-histidine, cyclo-arginyl-valine, cyclo-glutamyl-leucine, cyclo-leucyl-tryptophan, cyclo-tryptophanyl-tryptophan, cyclo-L-alanyl-proline, cyclo-methionyl-arginine, cyclo-lysyl-phenylalanine, cyclo-phenylalanyl-phenylalanine, cyclo-tryptophanyl-tyrosine, cyclo-asparaginyl-valine, cyclo-glutaminyl-isoleucine, cyclo-alanyl-serine, cyclo-methionyl-histidine, cyclo-methionyl-proline, cyclo-arginyl-leucine, cyclo-methionyl-glutamic acid, cyclo-methionyl-alanine, cyclo-isoleucyl-glutamic acid, cyclo-isoleucyl-serine, cyclo-valyl-serine, cyclo-methionyl-glycine, cyclo-valyl-threonine, and cyclo-valyl-aspartic acid, cyclo-glycyl-proline, cyclo-leucyl-proline, cyclo-glutaminyl-glycine, cyclo-tryptophanyl-lysine, cyclo-glutaminyl-phenylalanine, cyclo-lysyl-glycine, cyclo-seryl-lysine, cyclo-valyl-lysine, cyclo-asparaginyl-lysine, cyclo-histidyl-histidine, cyclo-threonyl-histidine, cyclo-aspartyl-histidine, cyclo-asparaginyl-histidine, cyclo-arginyl-serine, cyclo-asparaginyl-methionine, cyclo-glutaminyl-methionine, cyclo-tryptophanyl-arginine, cyclo-asparaginyl-arginine, cyclo-asparaginyl-proline, and cyclo-arginyl-arginine or salts thereof.

4. The GLP-1 secretion accelerator according to claim 3, wherein the cyclic dipeptide or salt thereof is three or more cyclic dipeptides selected from the group defined in claim 3 or salts thereof.

5. The GLP-1 secretion accelerator according to any one of claims 1 to 4, wherein the cyclic dipeptide including amino acids as constituent units is derived from soybean.

6. The GLP-1 secretion accelerator according to any one of claims 1 to 4, wherein the cyclic dipeptide including amino acids as constituent units is derived from tea.

7. Use of a cyclic dipeptide including amino acids as constituent units or a salt thereof for ameliorating carbohydrate metabolism, wherein the cyclic dipeptide or salt thereof is one or two or more cyclic dipeptides selected from the group consisting of
cyclo-threonyl-tyrosine, cyclo-valyl-tyrosine, cyclo-glutaminyl-tyrosine, cyclo-asparaginyl-isoleucine, cyclo-arginyl-proline, cyclo-asparaginyl-glycine, cyclo-methionyl-valine, cyclo-glutamyl-cysteine, cyclo-seryl-glutamic acid, cyclo-isoleucyl-lysine, cyclo-glutaminyl-leucine, cyclo-isoleucyl-threonine, cyclo-threonyl-threonine, cyclo-methionyl-threonine, cyclo-alanyl-cysteine, cyclo-glycyl-cysteine, cyclo-aspartyl-serine, cyclo-arginyl-aspartic acid, cyclo-glycyl-tryptophan, cyclo-histidyl-phenylalanine, cyclo-aspartyl-leucine, cyclo-isoleucyl-histidine, cyclo-seryl-leucine, cyclo-isoleucyl-aspartic acid, cyclo-seryl-cysteine, cyclo-phenylalanyl-tryptophan, cyclo-alanyl-leucine, cyclo-glutaminyl-histidine, cyclo-arginyl-valine, cyclo-glutamyl-leucine, cyclo-leucyl-tryptophan, cyclo-tryptophanyl-tryptophan, cyclo-L-alanyl-proline, cyclo-methionyl-arginine, cyclo-lysyl-phenylalanine, cyclo-phenylalanyl-phenylalanine, cyclo-tryptophanyl-tyrosine, cyclo-asparaginyl-valine, cyclo-glutaminyl-isoleucine, cyclo-alanyl-serine, cyclo-methionyl-histidine, cyclo-methionyl-proline, cyclo-arginyl-leucine, cyclo-methionyl-glutamic acid, cyclo-methionyl-alanine, cyclo-isoleucyl-glutamic acid, cyclo-isoleucyl-serine, cyclo-valyl-serine, cyclo-methionyl-glycine, cyclo-valyl-threonine, and cyclo-valyl-aspartic acid, cyclo-glycyl-proline, cyclo-leucyl-proline, cyclo-glutaminyl-glycine, cyclo-tryptophanyl-lysine, cyclo-glutaminyl-phenylalanine, cyclo-lysyl-glycine, cyclo-seryl-lysine, cyclo-valyl-lysine, cyclo-asparaginyl-lysine, cyclo-histidyl-histidine, cyclo-threonyl-histidine, cyclo-aspartyl-histidine, cyclo-asparaginyl-histidine, cyclo-arginyl-serine, cyclo-asparaginyl-methionine, cyclo-glutaminyl-methionine, cyclo-tryptophanyl-arginine, cyclo-asparaginyl-arginine, cyclo-asparaginyl-proline, and cyclo-arginyl-arginine or salts thereof.

8. The use according to claim 7, wherein the cyclic dipeptide or salt thereof is three or more cyclic dipeptides selected from the group defined in claim 7 or salts thereof.

9. The use according to claim 7 or 8, wherein the cyclic dipeptide including amino acids as constituent units is derived from soybean.

10. The use according to claim 7 or 8, wherein the cyclic dipeptide including amino acids as constituent units is derived from tea.
